(19) Europäisches Patentamt
European Patent Office
Office européen des brevets

(11) EP 0 362 771 B1

(12) # EUROPÄISCHE PATENTSCHRIFT

(45) Veröffentlichungstag und Bekanntmachung des Hinweises auf die Patenterteilung:
**31.05.2000 Patentblatt 2000/22**

(21) Anmeldenummer: **89118246.1**

(22) Anmeldetag: **02.10.1989**

(51) Int. Cl.$^7$: **C07D 491/107**, C07D 265/12, C07D 311/96, C07D 491/22 // (C07D491/107, 311:00, 221:00), (C07D491/107, 311:00, 241:00), (C07D491/107, 311:00, 239:00)

(54) **Verwendung von photochromen Substanzen**

Use of photochromic substances

Utilisation de substances photochromiques

(84) Benannte Vertragsstaaten:
**AT BE CH DE ES FR GB IT LI LU NL SE**

(30) Priorität: **01.10.1988 DE 3833436**

(43) Veröffentlichungstag der Anmeldung:
**11.04.1990 Patentblatt 1990/15**

(73) Patentinhaber:
**Optische Werke G. Rodenstock**
**80469 München (DE)**

(72) Erfinder: **Melzig, Manfred**
**D-8031 Wessling (DE)**

(74) Vertreter: **Münich, Wilhelm, Dr.**
**Dr. Münich & Kollegen**
**Anwaltskanzlei**
**Wilhelm-Mayr-Str. 11**
**80689 München (DE)**

(56) Entgegenhaltungen:
**EP-A- 0 246 114**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 362 771 B1

## Beschreibung

### Technisches Gebiet

Die Erfindung bezieht sich auf die Verwendung von photochromen Spiropyranen und Spiro-1,3-oxazinen.

Stand der Technik

Photochrome Spiropyrane sind für eine Vielzahl technischer Anwendungszwecke, u.a. Datenspeicherung, Energiekonversion und Lichtschutz, interessant. Dementsprechend zahlreich sind die in den letzten Jahren erschienenen Veröffentlichungen:

Die meisten dieser Veröffentlichungen beziehen sich auf Indolinobenzopyrane (US-PS 2 953 454, Berman), jedoch sind auch Strukturen wie Benzoxazolo- (US-PS 3 149 120, Berman) und Benzthiazolo- (US-PS 3 320 067, Taylor) sowie Benzodiazolo-Spiropyrane (FR-PS 14 51 332, Comp. de St. Gobain) einsetzbar. In der Literatur finden sich weitere Heteroatomringsysteme, wie Benzodithiolo- und Benzoxathiolospiropyrane (Gugliemetti, Helv. Chim. acta 58, 2563 (1975)) und Oxazolino- bzw. Thiazolidinospiropyrane (Gugliemetti, Bull. Soc. France, 568 (1978)).

Eine Spiroverknüpfung mit Heteroatom-6-Ringsystemen ist von Dibenzospiropyranen (US-PS 3 022 318, Berman) und ähnlichen benzoanellierten Verbindungen (US-PS 3 413 234, Taylor) her bekannt. Chinolinospiropyrane sind jedoch nicht mehr photochrom, da bei ihnen die Merocyaninstruktur die thermodynamisch stabilste Form darstellt (Wizinger, Helv.Chim.acta, 23, 247 (1940)).

Auch reine Kohlenwasserstoffgerüste, wie bei Adamantanospiropyranen (EP-A 0 246 114, Heller) können mit dem Pyranring verknüpft werden. Schließlich sind auch nicht spiroverknüpfte Benzo- und Naphthopyrane beschrieben (US-PS 3 567 605), die auch bei Raumtemperatur photochrome Verbindungen (EP-A 0 250 193, Heller) darstellen.

Photochrome Spirooxazine, insbesondere Indolinospironaphthoxazine sind ebenfalls seit langem bekannt (US-PS 3 562 172, Ono et al) und auch bereits in Sonnenschutzgläsern aus Kunststoff kommerziell in Einsatz (Rodenstock Perfalit Colormatic)

Die Patentschriften beinhalten jedoch nur substituierte Indolinospirobenz- und -naphthoxazine (PCT/DE 84/00275) oder solche, in denen eine oder mehrere CH-Gruppierungen in den aromatischen Ringen durch Stickstoff ersetzt sind (EP-A 0 141 407 (Kwak) und EP-A 0 245 020 (Rickwood)).

Bei dem Einsatz der vorgenannten Verbindungen beispielsweise in Sonnenschutzgläsern aus Kunststoffmaterial treten jedoch bei vielen dieser Verbindungen Probleme hinsichtlich Eindunkelungs- und Aufhellungsverhalten sowie der Lebensdauer des photochromen Effekts auf.

Erfindungsgemäß durchgeführte Untersuchungen an photochromen Benzo-und Naphthopyranen, die nicht spiroverknüpft sind, haben gezeigt, daß diese für den beabsichtigten Verwendungszweck nicht geeignet sind. Verbindungen, wie sie z.B. von Becker beschrieben werden, besitzen bei normalen Termperaturen (5-40°C) nicht nur in Lösung, sondern auch in Polydiethylenglykolbisallylcarbonat, einem für Brillengläser häufig verwendeten Kunststoffmaterial, eine derart schnelle Rückreaktion, daß die mit natürlichen Beleuchtungsstärken (10-100 Klux) erreichbaren Gleichgewichts-Konzentrationen der langweilig absorbierenden Form zu gering sind, um eine wahrnehmbare Eindunkelung bzw. Verfärbung zu erzeugen.

Die in der EP-A 0 250 193 beschriebenen Pyrane absorbieren im gewünschten Wellenlängenbereich zwischen ca. 450 und 700 nm, auch werden im Kunststoffmaterial bei Normaltemperaturen für Sonnenschutzzwecke ausreichende Einfärbungen erzielt. Die Lebensdauer ist jedoch auch unter Anwendung der dem heutigen Stand der Technik entsprechenden Schutzmaßnahmen (US-PS 4 720 356, Chu) völlig unzureichend. Bereits nach wenigen Eindunkelungs- und Aufhellungsvorgängen wird die Reaktion der mit diesen Verbindungen dotierten Gläser deutlich schwächer; zudem verleihen die entstehenden Photoprodukte diesen Gläsern eine zusätzliche irreversible Färbung.

Für eine kommerzielle Anwendung für Sonnenschutzzwecke sind - wie erfindungsgemäß erkannt worden ist - nur Pyrane geeignet, die in 2-Stellung über ein Spirokohlenstoffatom mit einem zweiten Zyklus verknüft sind. Einfache Ringsysteme, z.B. Cycloalkane ergeben jedoch nur bei Raumtemperatur nicht photochrome Verbindungen. Erst stark raumerfüllend substituierte Monozyklen und Polyzyklen sind unter bestimmten Voraussetzungen geeignet photochrom.

Einige Adamantanospiropyrane der EP-A 0 246 114 sind bei Normaltemperatur sichtbar photochrom, allerdings ist teilweise die Rückreaktion in die farblose Form für den angestrebten Verwendungszweck zu schnell und darüberhinaus die Temperaturempfindlichkeit des Effekts zu hoch.

Andererseits ergibt eine Substitution des H-Atoms in 3-Stellung des Pyranringes durch $-CH_3$, $-C_6H_5$ und -Br Verbindungen, die nicht photochrom sind. Eine Substitution des H-Atoms in 4-Stellung hingegen beschleunigt die Rückreaktion derart, daß die photochrome Einfärbung nur noch bei Temperaturen weit unter 0°C beobachtet werden kann.

Weiterhin tritt gerade beim Einsatz der vorgenannten Verbindungen in Sonnenschutzgläsern insbesondere aus Kunststoffmaterial folgendes Problem auf:

Zur Erzielung einer bestimmten Einfärbung, beispielsweise einer neutral grauen oder braunen Färbung ist es häufig erforderlich, mehrere unterschiedliche photochrome Substanzen, die in verschiedenen Wellenlängenbereichen absorbieren, zusammen in das Material des Sonnenschutzglases und/oder das Material eines auf dem Sonnenschutzglas aufgebrachten Schutzlackes insbesondere aus Polysiloxan aufzubringen. Damit ist es aber erforderlich, daß sich die unterschiedlichen Substanzen hinsichtlich ihres Eindunkelungs- und Aufhellungsverhalten sowie nach Möglichkeit auch hinsichtlich der Lebensdauer des photochromen Effekts möglichst "ähnlich" verhalten.

**Darstellung der Erfindung**

Der Erfindung liegt die Aufgabe zugrunde, die Verwendung für eine Reihe unterschiedlicher Verbindungen anzugeben, die in verschiedenen Wellenlängenbereichen absorbieren, und deren Eindunkelungs- und Aufhellungsverhalten ähnlich ist, so daß diese Verbindungen beispielsweise für Sonnenschutzgläser verwendbar sind und insbesondere die Verwendung mehrerer dieser Verbindungen gemeinsam in einem Sonnenschutzglas zur Erzielung einer bestimmten Farbe im eingedunkelten Zustand möglich ist.

Eine erfindungsgemäße Lösung dieser Aufgabe ist in den Patentansprüchen angegeben. Der Erfindung liegt dabei die im folgenden beschriebene Erkenntnis zugrunde, deren Anwendung auf die in den Ansprüchen angegebenen verwendeten Verbindungsklassen zu Verbindungen führt, deren Eindunkelungs- und Aufhellungsverhalten nicht nur ähnlich ist, sondern auch die Verwendung zweier oder mehrerer in unterschiedlichen Wellenlängenbereichen absorbierender Verbindungen in einem Sonnenschutzglas erlaubt.

Die erfindungsgemäße Einführung eines N-Atoms in 3-Stellung unter Bildung eines 1,3-Oxazinringes verbessert nicht nur das kinetische Verhalten beträchtlich, sondern bringt auch eine deutlich verbesserte Lebensdauer mit sich. Die Affinität des Pyranringes gegenüber einem oxidativen Angriff, der die lebensdauerbegrenzende Nebenreaktion photochromer Verbindungen dieser Klasse in Kunststoffgläsern darstellt, wird durch den elektronenziehenden Einfluß des Stickstoffatoms deutlich reduziert. Dieser Effekt wird in etwas abgeschwächter Form auch durch die Einführung von Stickstoffatomen in die dem Pyran(Oxazin)ring anellierten aromatischen Kerne erreicht. Die dadurch gebildeten Adamantanospirc-pyrido-, -pyrazino-, pyrimidino-, und -pyridazinopyrane(oxazine) und die höheren Homologen -chinolino-, -isochinolino-, -chinoxalino-, -chinazolino-, -phthalazino- und -phenanthrolinopyrane (oxazine) zeichnen sich darüber hinaus durch ein gegenüber reinen Kohlenstoffaromaten merklich besseres Färbeverhalten aus. Infolge des polareren Charakters sind bei der üblicherweise angewendeten Oberflächendiffusionsfärbung höhere Färbegeschwindigkeiten und/oder Konzentrationen des photochromen Farbstoffes möglich und damit stärker eindunkelnde Gläser herstellbar.

In diesem Sinne außerordentlich günstig wirkt es sich auch aus, wenn der Polyzyklus durch die Polarität und/oder Polarisierbarkeit erhöhende Gruppen substituiert wird. Dabei wirkt sich ähnlich wie bei elektronenziehend sübstituierten Adamantan die induktive Elektronenverarmung des Pyranringes lebensdauerverlängend aus.

Polyzyklen, die aufgrund ihrer Struktur oder Substitution, die bei der Ringöffnung nach Belichtung auf diesen Molekülteil übertragene positive Ladung zu stark stabilisieren, bilden die entgegengesetzte Grenze. Da dadurch die offene Form zur thermodynamisch begünstigten wird, liegen diese Verbindungen nur in der Merocyanform vor, eine Reversibilität der Einfärbung ist nicht mehr gegeben. Zumindest für die Kunststoffglasmaterialien Polymethylmethacrylat, und Polydiethylenglykolbisallylcarbonat ist die Sübstitution mit Nitrogruppen nicht empfehlenswert, da solche Verbindungen unter Lichteinwirkung mit der Matrix Nebenreaktionen eingehen, die zu einer gelbbraunen bis rotbraunen permanenten Verfärbung der Gläser führen.

Gegenstand der Erfindung ist die Verwendung von Adamantanospiroheteroaromatenpyranen

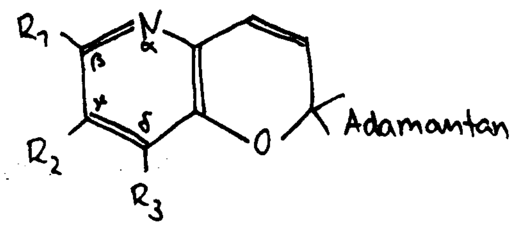

4 Pyridopyrane
(N-Atom in α, β, γ oder δ-Stellung)

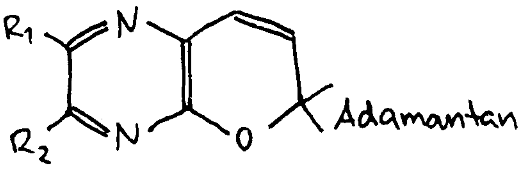

1 Pyrazinopyran

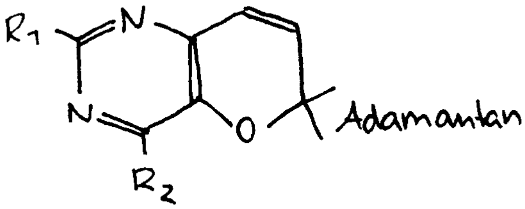

2 Pyrimidinopyrane
(N-Atome in α/γ oder β/δ-Stellung)

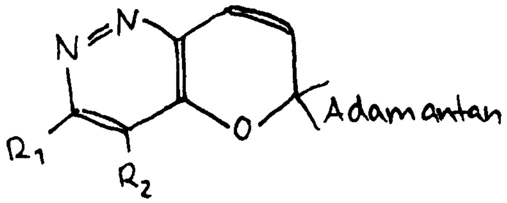

3 Pyridazinopyrane
(N-Atome in α/β, β/γ, γ/δ-Stellung)

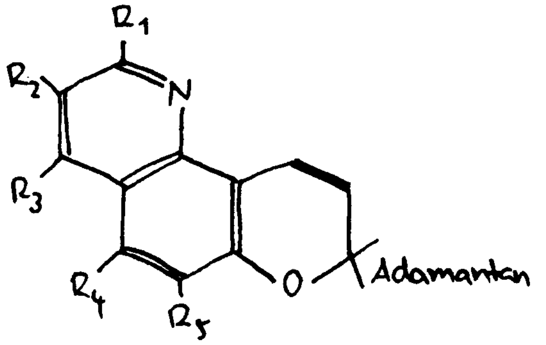

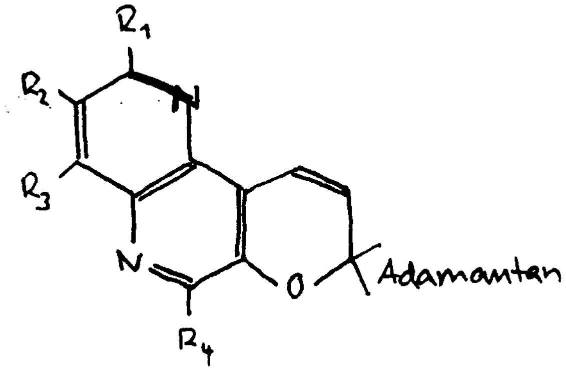

Chinolino-,
Isochinolino-,
Chinazolino-,
Chinoxalino-,
Phthalazino-,
Pteridino-,
Benzochinolino- und
Phenazinopyranen

wobei

| | |
|---|---|
| $R_1$ - $R_5$ : | ein Substituent aus der Reihe H, Alkyl, Naphthyl, OH, Alkoxy ($C_1$-$C_4$), Halogen, Alkylamino, Dialkylamino, Cyan und Trifluormethyl ist, oder |
| $R_1$ und $R_2$ oder $R_3$ und $R_4$ oder $R_2$ und $R_3$ | Bestandteil eines ankondensierten aromatischen oder heteroaromatischen Ringes oder eines Alkanringes mit 4-8 C-Atomen sind, |

als photochrome Substanz.

Ferner betrifft die Erfindung die Verwendung von Adamantanospiro(1.3)oxazinen der allgemeinen Formel

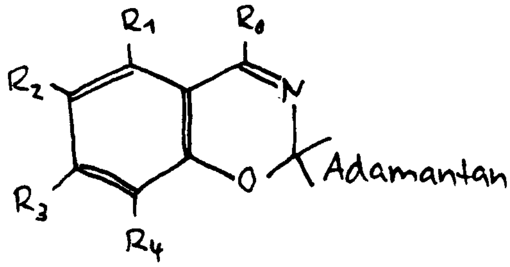

wobei

| | |
|---|---|
| $R_0$ | ein Substituent aus der Reihe H, Halogen, O-Alkyl, N-Alkyl, |
| $R_1$ - $R_4$ | ein Substituent aus der Reihe H, Alkyl, Naphthyl, OH, Alkoxy ($C_1$ - $C_4$), Halogen, Alkylamino, Dialkylamino, Cyan und Trifluormethyl ist, oder |
| $R_1$ und $R_2$ oder $R_3$ und $R_4$ oder $R_2$ und $R_3$ | Bestandteil eines ankondensierten aromatischen oder heteroaromatischen Ringes oder eines Alkanringes mit 4-8 C-Atomen sind, |

als photochrome Substanz.

Die Erfindung bezieht sich ferner auf die Verwendung von Spiro[Bicyclo[2.2.1]heptan-7,2'-4"-chlornaphtho[2,1-e]-(2H)-pyran] als photochrome Substanz.

Gegenstand der Erfindung ist auch die Verwendung von N-Heterocyclopyranen und Spirobenzo-, -naphtho-, -phenanthrooxazinen, dadurch gekennzeichnet, daß sie in 2-Stellung des Pyran- oder - oxazinringes ein substituiertes Adamantan, nämlich 1,3-Dimethyl- oder 1,3-Diazaadamantan, aufweisen mit den allgemeinen Formeln:

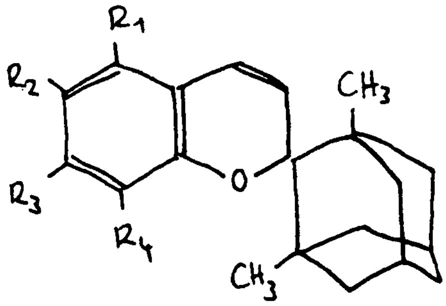

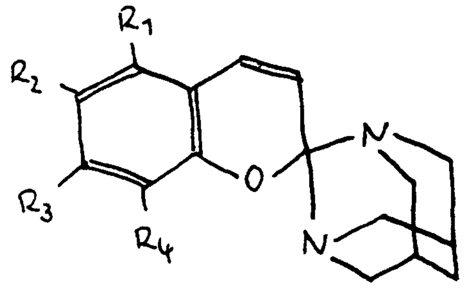

wobei die Reste

| | |
|---|---|
| $R_1$ - $R_4$ : | ein Substituent aus der Reihe H, Alkyl, Naphthyl, OH, Alkoxy ($C_1$ - $C_4$), Halogen, Alkylamino, Dialkylamino, Cyan und Trifluormethyl sind, oder |
| $R_1$ und $R_2$ oder $R_3$ und $R_4$ oder $R_2$ und $R_3$ | Bestandteil eines ankondensierten aromatischen oder heteroaromatischen Ringes oder eines Alkanringes mit 4-8 C-Atomen sind, |

als photochrome Substanz.

Die Erfingung betrifft auch die Verwendung von Spiro[adamantan-2,2'-(2H)-4'-chlorpyrido[4,3-e]-1',3'-oxazin], Spiro[1,3-Dimethyl-adamantan-2,2'-4''-chlornaphtho[2,1-e]-(2H)-pyran], Spiro[5,7-Dimethyl-adamantan-2,2'-4''-chlornaphtho[2,1-e]-(2H)-pyran], Spiro[1,3-Diazaadamantan-2,2'-4''-chlornaphtho[2,1-e]-(2H)-pyran] als photochrome Substanz.

Diese erfindungsgemäß verwendeten photochromen bzw. phototropen Substanzen weisen ein ähnliches Eindunkelungs- und Aufhellungsverhalten auf, das diese Verbindungen beispielsweise für Sonnenschutzgläser verwendbar macht.

## Beschreibung bevorzugter Ausführungsbeispiele

Im folgenden sollen verschiedene Beispiele für erfindungsgemäß verwendete Verbindungen besprochen werden:

Zur Herstellung der erfindungsgemäß verwendeten heterozyklenannellierten Spiro[adamantan-2,2'-(2H)-pyrane] sind prinzipiell mehrere Synthesewege geeignet.

Syntheseweg a): Umsetzung von Ethinyl-adamantylchlorid oder -acetat mit Aza-phenolen bzw. -naphtholen. Im folgenden ist schematisch und beispielhaft der Syntheseweg angegeben, wobei die Herstellung der in den einzelnen Schritten benötigten Verbindungen näher erläutert wird:

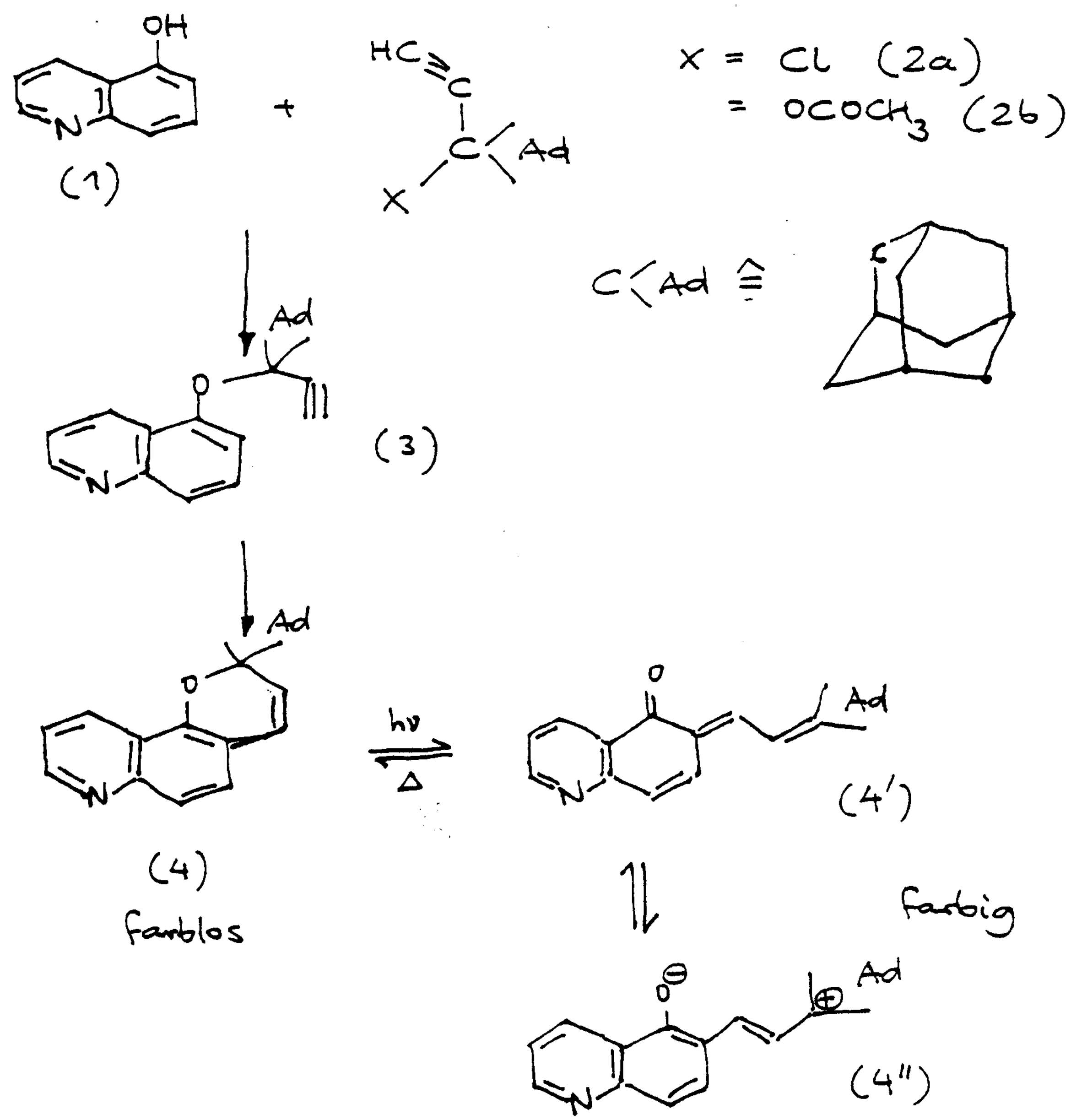


Im folgenden soll die Darstellung der einzelnen in dem vorstehenden Syntheseweg a) benötigten Verbindungen exemplarisch für das Beispiel 1 erläutert werden.

Darstellung der Verbindung (2b):

In einem mit Tropftrichter, Magnetrührer und Gaseinleitungsrohr versehenen 11 Dreihalskolben wird eine Lösung von 92 g Lithiumacetylid-ethylendiaminkomplex in 150 ml absoluten Dioxan vorgelegt. Durch die Lösung leitet man in leichtem Strom trockenes Acetylen. Über etwa 90 min werden 150 g Adamantanon in 450 ml absolutem Dioxan zugetropft. Die Tropfgeschwindigkeit wird so eingestellt, daß 60°C nicht überschritten werden. Nach beendetem Zutropfen wird bei ca. 55°C 15 h weitergerührt. Die erkaltete Lösung wird in eine Mischung aus 1 kg Eis und 300 ml 5% NaCl-Lösung gegossen. Mit verdünnter HCl wird der pH-Wert auf 5 eingestellt und zweimal mit je 250 ml Ether extrahiert. Der Extrakt wird mit $MgSO_4$ getrocknet und filtriert. Nach dem Abziehen des Ethers verbleiben 137 g eines weißlichen Feststoffs. Dieser besitzt nach dem Umkristallisieren aus Aceton/Hexan einen Schmelzpunkt von 102 -104 °C.
128 g des Ethinyl-adamantan-2-ols werden mit 60 ml Essigsäureanhydrid, 60 ml Pyridin und 32 ml Acetylchlorid 90 min unter Rückfluß gekocht, auf ein Gemisch von 750 g Eis und 750 g Aceton gegossen, mit Wasser auf 2l verdünnt und mit 3 mal 250 ml Ether extrahiert. Nach dem Trocknen mit $Na_2SO_4$, filtrieren und dem Abzug des Ethers verbleibt ein gelbstichiges Öl. Durch längeres Stehenlassen oder Verreiben mit etwas Pentan kristallisiert ein elfenbeinfarbener Feststoff aus, der nach dem Umkristallisieren (Aceton/Hexan) einen Schmelzpunkt von 64-65° C besitzt.

Dieser Feststoff besitzt folgende NMR-Daten:

$^1H$: δ: 2.64 (s,1H, ≡ CH); 2.05 (s, 3H, $CH_3$)

Darstellung der Verbindung (2a):

Die Darstellung des Ethinyl-adamantyl-2-ols erfolgt wie unter (2b) beschrieben.

37 g Ethinyl-adamantyl-2-ol werden in 200 ml trockenem Benzol gelöst. Unter Rühren und Kühlung auf 15-20°C wird Thionylchlorid in großem Überschuß zugetropft (250 ml). Anschließend wird unter Feuchtigkeitsausschluß noch 3h (Stunden) bei Raumtemperatur gerührt. Nach dem Abzug des Thionylchlorids und des Lösemittels am Rotavap verbleiben 34 g eines gelben Öls, das für die weitere Synthese ohne weitere Reinigung verwendet werden kann.

Darstellung der Verbindung (4):

In einem 2l Dreihalskolben mit Rührer und Tropftrichter und Rückflußkühler löst man 12g Na in 300 ml abs. EtOH und läßt in die Lösung 72 g 5-Hydroxy-chinolin (Produkt der Fa. Aldrich) in 600 ml abs. EtOH einlaufen. 100 g des Chlorids (2) werden mit 100 ml abs. EtOH verdünnt und anschließend unter kräftigem Rühren in die Alkoholatlösung getropft. Nach beendetem Zutropfen kocht man 5h unter Rückfluß. Die erkaltete Lösung wird mit 1l Eiswasser verdünnt und mit Ether extrahiert. Die organische Phase wird zuerst mit verd. NaOH, dann mit Wasser gewaschen, mit $Na_2SO_4$ getrocknet und filtriert. Dem Filtrat wird der Ether am Rotavap entzogen. Der Rückstand enthält den rohen Propargylether, d.h. die Verbindung (3) des vorstehenden Syntheseweges.

Die Umlagerung nach Imai und Ide (Chem.pharm. Bull. 10, 926 (1962)) erfordert hohe Drucke und Temperaturen, das in der EP-A 0 246 114 vorgeschlagene Verfahren mit saurem $Al_2O_3$ als Katalysator hohe Temperaturen. Diese führen zu einem hohen Prozentsatz nicht näher untersuchter Nebenprodukte. Erfindungsgemäß wird daher das Verfahren nach Koch-Pomeranz et al. (Helv. chim.acta. 56, 2981 (1973)) bevorzugt:

80 g des Propargylethers (3) und 25 g Silbertetrafluoroborat, gelöst in 500 ml Chloroform werden 8 h bei Raumtemperatur gerührt. Nach dem Ausschütteln mit 10 proz. wässrigen KCN-Lösung wird die organische Phase mit $Na_2SO_4$ getrocknet. Der nach dem Abziehen des Lösemittels verbleibende Rückstand wird mit Toluol an $Al_2O_3$ chromatographiert. Es verblieben 9,2 g Spiro [adamantan-2,2′-(2H)-pyrano[6,5-f] chinolin], d.h. die mit (4) bezeichnete Verbindung. Nach dem Umkristallisieren aus $CHCl_3/C_6H_{12}$ erhält man einen hellsandfarbenen Feststoff, der einen Schmelzpunkt (Smp) von 246°C hat. Die belichtete Lösung in $CH_2Cl_2$ zeigt eine orange Farbe ($\lambda_{max}$ = 442 nm).

In gleicher Weise wurden die als Beispiele 2 angeführten Verbindungen aus den im Handel erhältlichen Hydroxy-azaaromaten hergestellt. Bei den folgenden Beispielen ist zunächst die eingesetzte Ausgangsverbindung und dann die erhaltene Endverbindung angegeben.

Beispiel 1: 5-Hydroxychinolin (Ausgangsverbindung) Spiro(adamantan-2,2′-(2H) pyrano [6,5-f] chinolin), Smp.: 246°C

Beispiel 2: 4-Hydroxychinolin (Ausgangsverbindung) Spiro[adamantan-2,2′-(2H) pyrano [5,6-C] chinolin], Smp.: 219°C

Beispiel 3: 8-Hydroxychinolin (Ausgangsverbindung) Spiro[adamantan-2,2′-(2H) pyrano [5,6-h] chinolin], Smp.: 223°C

Beispiel 4: 2-Hydroxychinoxalin (Ausgangsverbindung) Spiro[adamantan-2,2′-(2H) pyrano [5,6-b]-chinoxalin], Smp: 151°C

Beispiel 5: 4-Hydroxychinaldin (Ausgangsverbindung) Spiro[adamantan-2,2′-(2H) pyrano [5,6-c] 2''-methyl-chinolin], Smp.: 179°C

Beispiel 6: 8-Hydroxychinaldin (Ausgangsverbindung) Spiro[adamantan-2,2′-(2H) pyrano [5,6-h] 2''-methylchinolin], Smp.: 162°C

Beispiel 7: 5-Hydroxyisochinolin (Ausgangsverbindung) Spiro[adamantan-2,2′-(2H) pyrano [6,5-f] isochinolin],

Smp.: 229° C

Beispiel 8: 2-Hydroxypyridin (Ausgangsverbindung) Spiro[adamantan-2,2′-(2H) pyrano [6,5-b] pyridin], Smp.: 154°C (Z.)

Beispiel 9: 3-Hydroxypyridin (Ausgangsverbindung) Spiro[adamantan-2,2′-(2H) pyrano [6,5-c] pyridin], Smp.: 178°C

Beispiel 10: 4-Hydroxypyridin (Ausgangsverbindung) Spiro[adamantan-2,2'-(2H) pyrano [5,6-c] pyridin], Smp.: 166°C

Beispiel 11: 4-Hydroxypyrimidin (Ausgangsverbindung) Spiro[adamantan-2,2'-(2H) pyrano [6,5-d) pyrimidin], Smp.: 142°C (Z.)

Beispiel 12: 3-Hydroxy-6-methyl-pyridin (Ausgangsverbind.) Spiro[adamantan-2,2'-(2H) pyrano [6,5-c] 6"-methyl-pyridin, Smp.: 139°C (Z.)

(Z.: Zersetzung)

Die o-Hydroxy-aza-aromaten der Beispiele 1,4,5,8,10 und 11 liegen teilweise in der tautomeren -on-Form (1H-Pyridon etc.) vor. Dies erschwert die Bildung der Propargylether.

Insgesamt weist der vorstehend beschriebene Syntheseweg trotz der leichten Zugänglichkeit der Ausgangsprodukte (1) Nachteile auf, da die Ausbeuten an der Verbindung (3) zum Teil sehr gering sind (≤ 10%). Versuche mit Ethinyl-adamantylacetat als Verbindung (2b) analog den Beispielen 16 und 17 der EP-A 0 246 114 ergaben i.a. noch schlechtere Resultate.

Syntheseweg b): Umsetzung der o-Acetyl-hydroxy-aza-aromaten mit Adamantanon, anschließende Reduktion und Dehydratisierung zum 2H-Pyran.

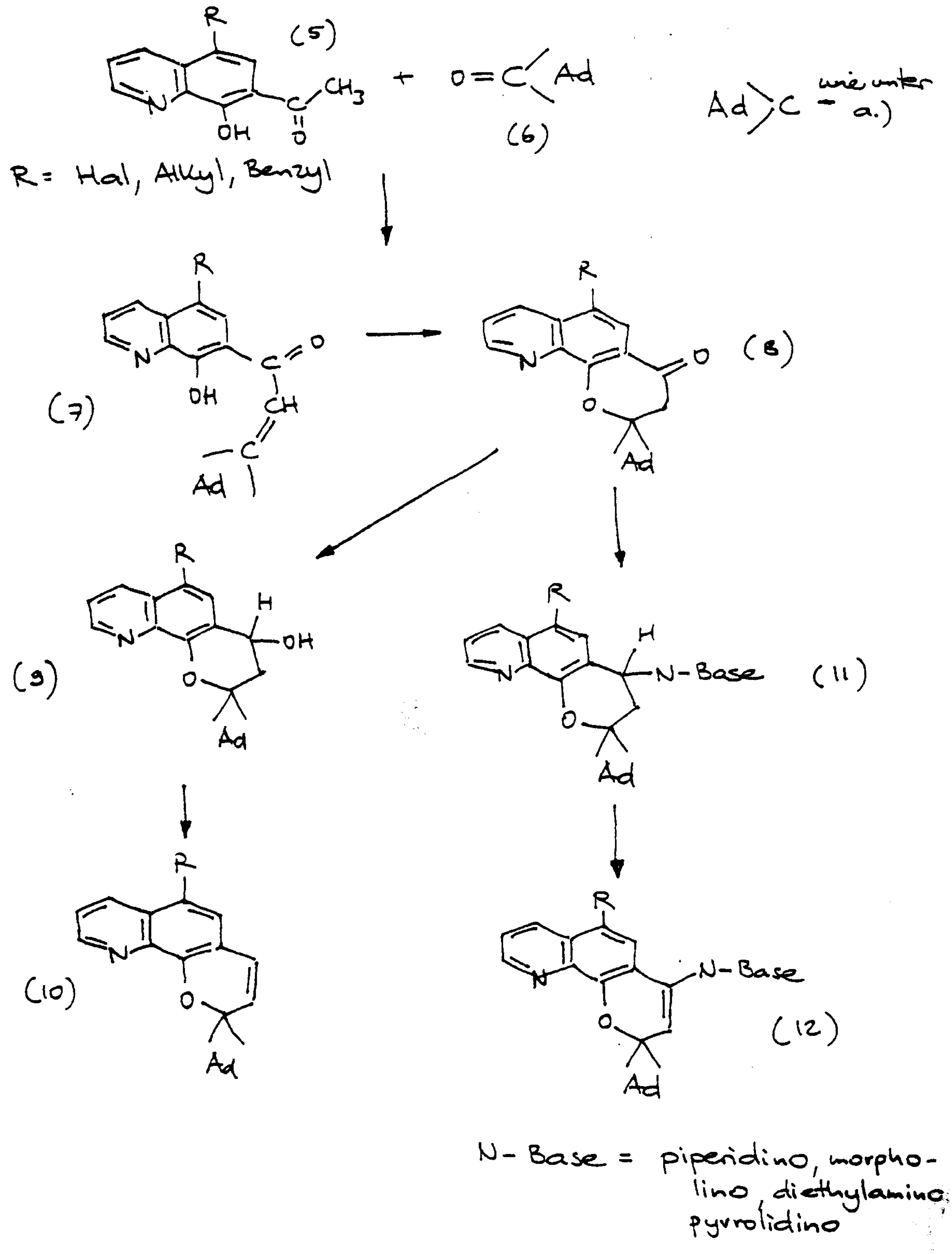


Im folgenden soll die Darstellung der einzelnen in dem vorstehenden Syntheseweg b) benötigten Verbindungen exemplarisch für das Beispiel 13 erläutert werden.

Darstellung der Verbindung (5):

Die verwendeten o-Acetyl-hydroxy-aza-aromaten sind nach bekannten Methoden (vgl. Beispiele 13-21) in meist guten Ausbeuten zugänglich.

Darstellung der Verbindung (8):

50 g 7-Acetyl-8-hydroxy-5-methyl-chinolin und 50 g Adamantanon wurden mit 250 ml Toluol und 20 ml Pyrrolidin 3h unter Rückfluß gekocht. Das gebildete Reaktionswasser wird am Wasserabscheider entfernt. Der schwarzbraunen Lösung wird am Rotavap das Toluol entzogen. Der ölige Rückstand wird mit 2 ml conc. HCl in 200 ml Methanol durchgeschüttelt. Nach dem Abziehen des Methanols verbleiben 76 g eines dunkelbraunen Öls, nämlich der Verbindung (8).

Darstellung der Verbindung (9):

In einem 500 ml Dreihalskolben mit Magnetrührer und Rückflußkühler wurden 15 g der Verbindung (8) in 300 ml n-Butanol gelöst und 30 g $NaBH_4$ in kleinen Mengen zugegeben. Nach beendeter Zugabe wird noch 3 h gekocht. Nach dem Abzug des Butanols wird der Rückstand in 250 ml $H_2O$ aufgenommen und mit $CH_2Cl_2$ extrahiert. Der organische Extrakt wird mit $MgSO_4$ getrocknet und filtriert. Nach dem Abzug des $CH_2Cl_2$ verbleiben 8g eines schwarzen Öls. Dieses wird an neutralem $Al_2O_3$ mit $CH_2Cl_2$/Hexan (von 1:5 auf 5:2 steigend) chromatographiert. Aus der Hauptfraktion lassen sich nach Abzug der Eluierungsmittel 6,1 g eines ockerfarbenen Feststoffes, nämlich der Verbindung (9) gewinnen.

Darstellung der Verbindung (10):

6 g der Verbindung (9) werden in einem Rundkolben vorsichtig mit 3 g wasserfreiem $CuSO_4$ eingeschmolzen und ca. 15 min. als Schmelze erhalten. Es empfiehlt sich einen Luftkühler aufzusetzen, da unter den geg. Bedingungen die Substanz (10) teilweise sublimiert. Kühler und abgekühlte, fein gemahlene Schmelze werden mit $CHCl_3$ gewaschen bzw. extrahiert. Der org. Extrakt wird mit $MgSO_4$ getrocknet und filtriert. Nach dem Abziehen des Lösemittels verbleiben 4,6 g eines orangegelben Öls. Dieses wird wie obenstehend bei der Darstellung der Verbindung (9) beschrieben chromatographiert. Es verbleiben 3,9 g einer hellgelben Substanz mit dem Schmelzpunkt 214°C, nämlich der Verbindung 10.

Die Beispiele 14-21 werden in analoger Weise hergestellt.

Beispiel 13: 7-Acetyl-8-hydroxy-5-methyl-chinolin (Ausgangsverb. (5)) E. Hodel, US-PS 3 113 135 Spiro[adamantan-2,2'-(2H) pyrano-[5,6-h]-5''-methylchinolin], Smp.: 214°C

Beispiel 14: 7-Acetyl-8-hydroxy-5-chlor-chinolin (wie 13) Spiro[adamantan-2,2'-(2H) pyrano-[5,6-h]-5''-chlorchinolin], Smp.: 182°C

Beispiel 15: 7-Acetyl-8-hydroxy-5-benzyl-chinolin (wie 13) Spiro[adamantan-2,2′-(2H) pyrano-[5,6-h]-5''-benzylchinolin], Smp.: 133°C (Z.)

Beispiel 16: 7-Acetyl-8-hydroxy-5-chlor-chinaldin (wie 13) Spiro[adamantan-2,2′-(2H) pyrano-[5,6-h]-5''-chlor-2''-methylchinolin, Smp.: 166°C (Z.)

Beispiel 17: 7-Acetyl-8-hydroxy-5-methyl-chinaldin (wie 13) Spiro[adamantan-2,2′-(2H) pyrano-[5,6-h]-2'',5''-dimethylchinolin, Smp.: 174°C (Z.)

Beispiel 18: 3-Acetyl-4-hydroxy-chinolin F. Kroenke e al., Ann. 644, 93 (1961); die erhaltene Substanz ist identisch mit der aus Beispiel 2

Beispiel 19: 4-Acetyl-3-hydroxy-pyridin
W.H. Hunter, DE-A 2 014 779
erhaltene Substanz identisch mit der aus Beispiel 9

Beispiel 20: 2-Acetyl-3-hydroxy-pyridin
nach T. Yamazaki et al., Chem. Pharm.Bull, 1150 (1977)

Spiro[adamantan-2,2′-(2H) pyrano-[5,6-6] pyridin]
Smp.: 150°C

Beispiel 21: 3-Acetyl-4-hydroxy-6-methyl-chinolin
M.S. Mayadeo, Ind. J. Chem. 599 (1984)
Spiro[adamantan-2,2′-(2H)-pyrano-[5,6-c]-6''-methylchinolin]
Smp.: 227-229°C

Der Einsatz von annähernd äquimolaren oder größeren Mengen sekundären Amins als Kondensationshilfsmittel bei der Reaktion (7) -> (8) führt zu 4-substituierten Pyranon (II) als Hauptprodukt. Diese lassen sich in gleicher Weise wie die 4-Hydroxypyrane (Verbindung (9)) dehydratisieren.

Dabei haben die nachfolgend angegebenen, durch Stickstoffbasen substituierten 2H-Pyrane eine schlechtere Lebensdauer verglichen mit den unsubstituierten Verbindungen.

Beispiel 22: Spiro[adamantan-2,2'-4'-pyrrolidino-(2H) pyrano[5,6-h]-5''-methylchinolin]
Smp.: 226°C (Z.)

Beispiel 23: Spiro[adamantan-2,2'-4'-piperidino-(2H) pyrano[5,6-h]-5''-methyl-chinolin]
Smp.: 217°C (Z.)

Beispiel 24: Spiro[adamantan-2,2'-4'-piperidino-2(H) pyrano[5,6-h]-5''-chlorchinolin]
Smp.: 169°C (Z.)

Als nächstkommender Stand der Technik für die nachfolgend aufgeführten Untersuchungen wurden die Substanzen des Beispiels 1,4 und 14 der EP-A 0 246 114 nach den dort beschriebenen Angaben synthetisiert.

Vergleich 1: Spiro[adamantan-2,2'-(2H) benzopyran]
Vergleich 2: Spiro[adamantan-2,2'-4''-chlor-naphtho [2,1-e](2H)pyran]
Vergleich 3: Spiro[adamantan-2,2'-4'-piperidino-(2H)benzopyran]

Die erfindungsgemäß verwendeten Verbindungen und die Vergleichssubstanzen wurden in einem Lack gemäß DE-OS 3 156 568 (in einer Konzentration von 4%) zur Einfärbung von 2mm dicken Plangläsern aus Polydiethylenglykolbisallylkarbonat verwendet. Dieses Material, auch unter dem Handelsnamen CR-39 der Fa. PPG bekannt, ist das weltweit am häufigsten verwendete Polymer für Kunststoffgläser.

Die so hergestellten Gläser wurden spektral vermessen. Kennwert ist die optische Dichte an der Stelle des langwelligen Absorptionsmaximums $\lambda_{max}$ (ca. 420 - 470 nm) eines nach DIN 58217 mit 60 Klux bei 23°C 15 Minuten belichteten Glases. Zur Erreichung des unbelichteten Zustandes wurden die Gläser bei 85-90°C 30 Minuten ausgeheizt, 30 Minuten im Dunkeln abkühlen gelassen und dann vermessen.

$$\Delta OD = OD\lambda_{max}\ (belichtet) - OD\lambda_{max}\ (unbelichtet)$$

Die Gläser wurden in einem Bestrahlungstestgerät (Suntest der Fa. Original Hanau) bei 40°C mit ca. 130 Klux belichtet, bis die Reaktion des Glases OD auf 1/e des ursprünglichen Wertes zurückgegangen war. Die so ermittelte Bestrahlungszeit LD ist ein Maß für die Lebenserwartung des photochromen Effekts eines Glases im normalen Gebrauch.

$$\Delta OD\ (o) = e\ {}^*\Delta OD\ (LD)$$

Tabelle 1

| Beispiel | ΔOD (o) | LD (h) |
|---:|---|---|
| 1 | 0.624 | 26.2 |
| 2 | 0.314 | 35.1 |
| 3 | 0.472 | 22.2 |

Tabelle 1 (fortgesetzt)

| Beispiel | ΔOD (o) | LD (h) |
|---|---|---|
| 5 | 0.265 | 28.4 |
| 6 | 0.392 | 15.8 |
| 7 | 0.577 | 25.7 |
| 9 | 0.332 | 12.2 |
| 10 | 0.308 | 13.4 |
| 12 | 0.250 | 16.9 |
| 14 | 0.668 | 24.9 |
| 15 | 0.281 | 11.4 |
| 16 | 0.252 | 15.2 |
| 20 | 0.279 | 23.3 |
| 21 | 0.419 | 23.7 |
| 22 | 0.316 | 7.1 |
| 24 | 0.298 | 6.4 |
| Vergleich 1 | 0.459 | 8.4 |
| Vergleich 2 | 0.644 | 14.2 |
| Vergleich 3 | 0.433 | 2.6 |

Deutlich erkennbar ist die Überlegenheit der erfindungsgemäß verwendeten Substanzen bezüglich der Lebensdauer des photochromen Effekts. Die in 4-Stellung mit einer Stickstoffbase substituierten 2H-Pyrane (Beispiel 22-24) sind in der Lebensdauer deutlich schlechter, den direkt vergleichbaren Verbindungen nach dem Stand der Technik (Vergleich 3) jedoch überlegen.

Durch die Einführung von Stickstoffatomen in dem (2H)-Pyranring annellierten Benzo- bzw. Naphtho-Ringsystem wird die Elektronendichte in diesem herabgesetzt. Stickstoffbasen in 4-Stellung des (2H)-Pyranrings erhöhen dessen Elektronendichte. Dies erhöht bzw. verringert, wie erfindungsgemäß nachgewiesen, die Lebensdauer des photochromen Effekts.

Noch wirkungsvoller ist die Einführung eines Stickstoffatoms in den 2H-Pyranring selbst.

Im folgenden soll die Synthese des (2H)1,3-Benzoxazin-Grundgerüsts beschrieben werden. Hierzu geht man von einem Salicylsäureamid (13) und Adamantanon (6) aus (vgl. H.O.L. Fischer, Ber. 65, 1032 (1932)). Je nach Weiterreaktion sind verschiedenartig 4'-substituierte Spiro[adamantan-2,2'-(2H)-1'.3'-benzoxazine] synthetisierbar.

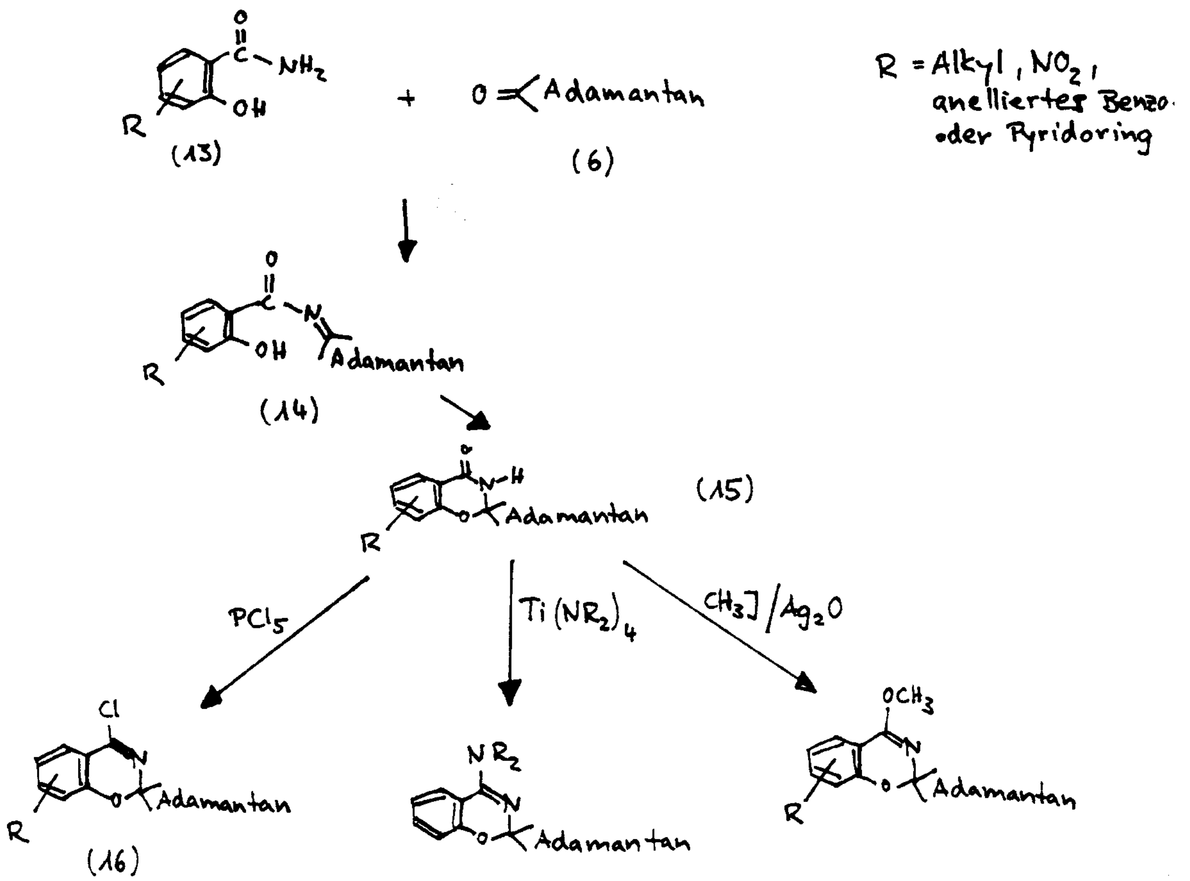


Im folgenden soll die Darstellung der einzelnen in dem vorstehenden Syntheseweg benötigten Verbindungen exemplarisch für das Beispiel 25 erläutert werden.

Darstellung der Verbindung (15):

In einem 500 ml Kolben werden 13,7 g Salicylamid und 16,8 g Adamantanon in 150 ml Chloroform zu 25 g Polyphosphatethylester gegeben und unter Rühren 5 h unter Rückfluß gekocht. Nach dem Abzug der Lösemittel wird der Rückstand mit 10% wässriger NaOH hydrolysiert. Die dabei ausfallenden Kristallmassen werden abgesaugt, gemahlen und zur vollständigen Hydrolyse in 500 ml 8% wässriger NaOH von 35°C eingerührt. Nach dem Absaugen wird mit Wasser nachgewaschen. Das Produkt wird aus Aceton/Hexan umkristallisiert. Man erhält hellgelbe Kristalle mit einem Fp. 220-225° C (Z.), nämlich die Verbindung (15).

Darstellung von (16):

12,9 g (15) werden in 200 ml absoluten Benzol gelöst und unter Rühren während 10 min. 10,5 g $PCl_5$ zugetropft. Anschließend wird noch 15 min. bei 75°C gerührt. Nach dem Abziehen des $POCl_3$ und des Lösemittels verbleibt das Imidoylchlorid als gelbbraunes Öl. Dieses Öl wird durch Chromatographie an neutralem $Al_2O_3$ mit Toluol/Hexan (2:1) als Eluierungsmittel gereinigt. Man erhält hellockerfarbene Kristalle mit einem Fp. von 119°C, nämlich die Verbindung (16).

Durch die Verwendung von substituierten O-Hydroxybenzoe bzw- naphthoesäure-amiden lassen sich weitere Verbindungen der gleichen Klasse synthetisieren. Werden 3-Hydroxyisonicotin oder 5-Nitrosalicyl-säureamid als Ausgangsstoffe verwendet, so läßt sich die Elektronendichte im Oxazinring weiter verringern.

Beispiel 25: Saliylsäureamid (Ausgangsverbindung 13) Spiro[adamantan-2,2′-(2H)-4′-chlor-1,3′-benzoxazin](Produktverbindung (17))
Smp.: 119°C

Beispiel 26: m-Kresotinsäureamid (Ausgangsverbindung 13) Spiro [adamantan-2,2′-(2H)-4′-chlor-7′-methyl-1′.3′-benzoxazin] (17)
Smp.: 114°C

Beispiel 27: 1-Hydroxy-2-naphthoesäureamid (13) Spiro[adamantan-2,2′-(2H)-4′-chlor-naphtho [2,1-e]-1′,3′-oxazin] (17)
Smp.: 136°C

Beispiel 28: 3-Hydroxyisonicotinsäureamid (13) Spiro[adamantan-2,2′-(2H)-4′-chlor-pyrido [4,3-e]-1′.3′-oxazin (17)
Smp.: 152° C

Beispiel 29: 5-Nitrosalicylsäure (Ausgangsverbindung 13) Spiro[adamantan-2,2′-(2H)-4′-chlor-6′-nitro-1′.3′-benzoxazin] (17)
Smp.: ≈ 130° C (Z.)

Wird die Verbindung (15) analog dem von J.D. Wilson beschriebenen Verfahren (J. Org.Chem 36, 1613 (1971)) mit Tetrakisdialkylaminotitan (Käufl.) umgesetzt, so erhält man die zyklischen Amidine (Verbindung 17).

Die Umsetzung der Verbindung (15) mit Alkyljodid/$Ag_2O$ in Dioxan analog H.O.L. Fischer (Ber. 65, 1032 (1932)) liefert die entsprechenden 4-alkoxy substituierten Verbindungen (18).

Da die Verbindungen (17) und (18) gegenüber den analogen Verbindungen (16) eine höhere Elektronendichte im Oxazinring und dadurch eine geringere Lichtstabilität aufweisen, wurden sie als weniger bevorzugte Verbindungen nicht in die Ergebnisse der Tabelle 2 einbezogen. Diese enthält die Werte der mit den Substanzen der Beispiele 25-29 in gleicher Weise wie vorstehend beschrieben gefärbten und einem Lebensdauertest unterworfenen Gläser.

Tabelle 2

| Beispiel | ΔOD (o) | LD (h) |
|---|---|---|
| 25 | 0.304 | 43.4 |
| 26 | 0.236 | 36.2 |
| 27 | 0.277 | 31.8 |
| 28 | 0.228 | 42.6 |
| 29 | 0.416 | 7.4* |

Die teilweise auftretende bleibende Braunfärbung der Gläser dürfte durch eine Reaktion der Nitrogruppe mit der Kunststoffmatrix entstehen.

Während die vorgenannten Änderungen am Pyranringsystem einen großen Einfluß auf die Lebensdauer des photochromen Effekts besitzen, ist ihr Einfluß auf die Kinetik der Aufhellreaktion, also der Rückreaktion, bei der der Pyranring wieder geschlossen wird, eher gering.

Bereits kleinere Modifikationen am Adamantanteil verändern jedoch die Aufhellgeschwindigkeiten drastisch.

Dies wird im folgenden beschrieben:

Die Darstellung der substituierten Adamantanone oder Norbornanone erfolgt nach den in der Literatur ersichtlichen Angaben. Die weitere Synthese kann nach einem der eingangs erläuterten Wege a oder b erfolgen.

Adamantan-dione (harzige Produkte) und Thiaadamantane (instabil unter den Reaktionsbedingungen) konnten nach keinem der Wege a und b zu photochrome Verbindungen umgesetzt werden.

Die Synthese der Beispiele 30-33 erfolgte mit 2-Acetyl-4-chlor-1-hydroxy-naphthalin nach Route b.

Entsprechend den Arbeiten von I.N. Azerbaev (Vses.Konf. Khim Atsetilena 5th 268, (1975)) und S.A. Baisalbaeva (Deposited Doc. 1983, VINITI 1898-1983), die hochsubstituierte Ethynyl-adamant-6-ole hergestellt haben, ist jedoch auch der Syntheseweg b wählbar.

Es ist ohne weiteres ersichtlich, daß die Azaphenole und -naphthole der vorherigen Beschreibung mit den im folgenden aufgeführten Polyzyklen kombiniert werden können.

Auch bei den folgenden Beispielen ist wiederum zunächst die Ausgangsverbindung und dann die erhaltene Endverbindung angegeben.

Beispiel 30: 1,3-Dimethyl-adamantan-2-on

oder: 1,3-Dimethyl-tricyclo[3.3.1.1$^{3,7}$]-decan-2-on nach D. Lenoir et al., J.A.C.S. 96, 2157 (1974) Spiro [1,3-Dimethyl-adamantan-2,2′-4"-chlornaphtho[2,1-e] (2H)-pyran]
Smp.: 124-127°C (Cyclohexan)

Beispiel 31: 5,7-Dimethyl-adamantan-2-on (wie 30) Spiro [5,7-Dimethyl-adamantan-2,2′-4"-chlornaphtho[2,1-e] (2H)-pyran]
Smp.: 141-144°C (Aceton/Pentan)

Beispiel 32: 1,3-Diaza-adamantan-2-on
J. Kuthan et al., Coll. Czech.Chem. Comm. 38, 3491 (1973)
Spiro [1,3-Diazaadamantan-2,2′-4"-chlornaphtho[2,1-e](2H)-pyran]
Smp.: 149°C (Z)

Beispiel 33: Norbornan-7-on

oder: Bicyclo[2.2.1]heptan-7-on
P.G. Gassmann, Tetrahedron Letters 9 (1963) Spiro-[Bicyclo[2.2.1]heptan-7,2′-4"-chlornaphtho [2,1-e](2H)-pyran]
Smp.: 120-124°C (Cyclohexan)

Mit den Substanzen der Beispiel 30-33 und den Vergleichssubstanzen 1 und 2 wurden, wie zuvor angegeben, Kunststoffgläser eingefärbt.

An diesen Gläsern wurde bei $\lambda_{max}$ der langwelligen Absorption nach einer 15-minütigen Belichtung mit 60 Klux bei 23°C der Verlauf der Rückreaktion (4" bzw. 4' -> 4) beobachtet.

Gewertet wurde die Zeit, die vergeht, bis ΔoD auf 1/e des ursprünglichen Wertes zurückgeht.

| Beispiel | Zeit t (min) |
|---|---|
| 30 | 214 |
| 31 | 38 |
| 32 | 14 |
| 33 | 3,6 |
| Vergleich 1 | 32 |
| Vergleich 2 | 26 |

Die Substitution der beiden CH-Gruppierungen, die dem Spiro-C-Atom des Adamantans benachbart sind, durch C-$CH_3$ behindert die Rückreaktion sehr stark. Substitutionen in 5,7-Stellung haben einen wesentlich geringeren Einfluß.

Die Substitution durch N beschleunigt die Rückreaktion. Der Effekt wird möglicherweise durch die Stabilisierung der ionischen Form 4" (durch teilweise Übernahme der formalen positiven Ladung durch die Stickstoffatome) verringert.

Der Ersatz des sperrigen Adamantanrestes durch den wesentlich kleineren Norbornanrest bringt eine mehrfach schnellere Aufhellung der Einfärbung mit sich.

## Patentansprüche

1. Verwendung von Adamantanospiroheteroaromatenpyranen

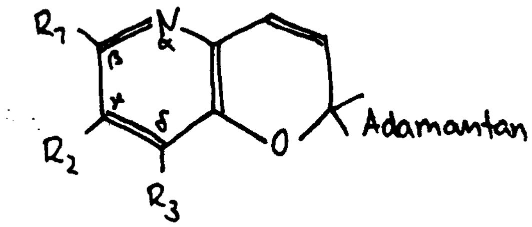

4 Pyridopyrane
(N-Atom in α, β, γ oder δ-Stellung)

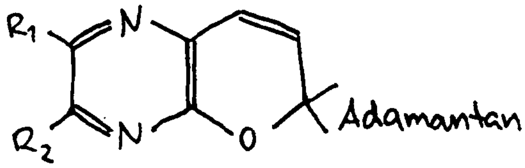

1 Pyrazinopyran

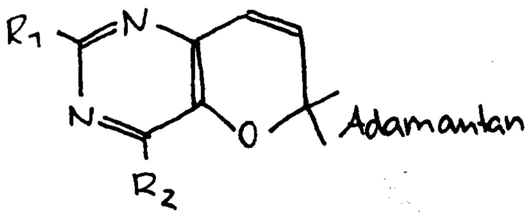

2 Pyrimidinopyrane
(N-Atome in α/γ oder β/δ-Stellung)

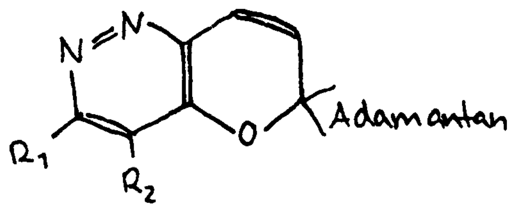

3 Pyridazinopyrane
(N-Atome in α/β, β/γ, γ/δ-Stellung)

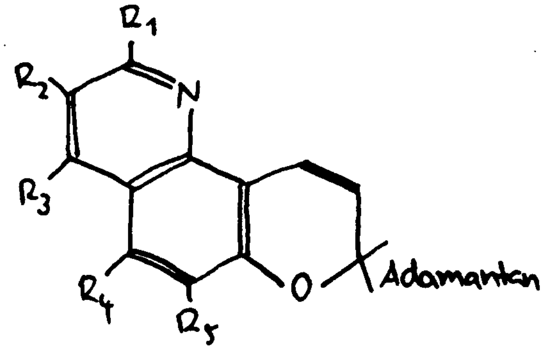

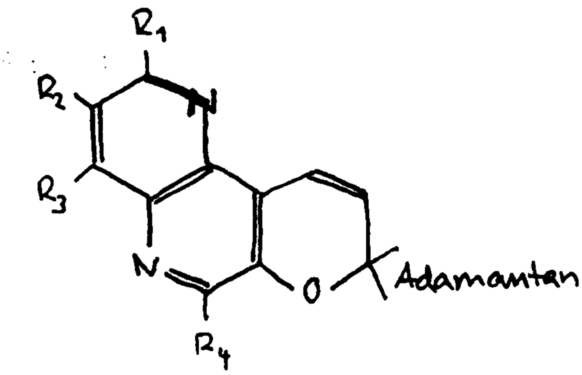

Chinolino-,
Isochinolino-,
Chinazolino-,
Chinoxalino-,
Phthalazino-,
Pteridino-,
Benzochinolino- und
Phenazinopyranen

wobei

$R_1$ - $R_5$ : ein Substituent aus der Reihe H, Alkyl, Naphthyl, OH, Alkoxy ($C_1$-$C_4$), Halogen, Alkylamino, Dialkylamino, Cyan und Trifluormethyl ist, oder

$R_1$ und $R_2$ oder $R_3$ und $R_4$ oder $R_2$ und $R_3$ Bestandteil eines ankondensierten aromatischen oder heteroaromatischen Ringes oder eines Alkanringes mit 4-8 C-Atomen sind,

als photochrome Substanz.

**2.** Verwendung von Adamantanospiro(1.3)oxazinen der allgemeinen Formel

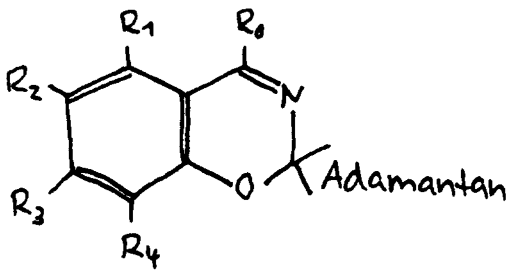

wobei

$R_0$ ein Substituent aus der Reihe H, Halogen, O-Alkyl, N-Alkyl,

$R_1$ - $R_4$ ein Substituent aus der Reihe H, Alkyl, Naphthyl, OH, Alkoxy ($C_1$ - $C_4$), Halogen, Alkylamino, Dialkylamino, Cyan und Trifluormethyl ist, oder

$R_1$ und $R_2$ oder $R_3$ und $R_4$ oder $R_2$ und $R_3$ Bestandteil eines ankondensierten aromatischen oder heteroaromatischen Ringes oder eines Alkanringes mit 4-8 C-Atomen sind,

als photochrome Substanz.

**3.** Verwendung von Spiro[Bicyclo[2.2.1]heptan-7,2'-4''-chlornaphtho[2,1-e]-(2H)-pyran] als photochrome Substanz.

**4.** Verwendung von N-Heterocyclopyranen und Spirobenzo-, -naphtho-, -phenanthrooxazinen, dadurch gekennzeichnet, daß sie in 2-Stellung des Pyran- oder -oxazinringes ein substituiertes Adamantan, nämlich 1,3-Dimethyl- oder 1,3-Diazaadamantan, aufweisen mit den allgemeinen Formeln:

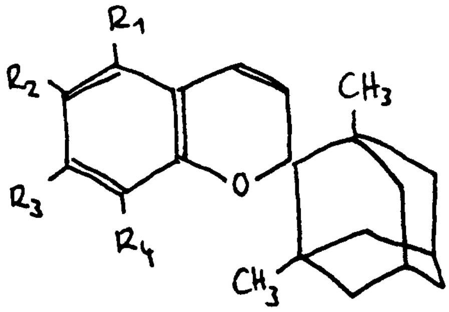

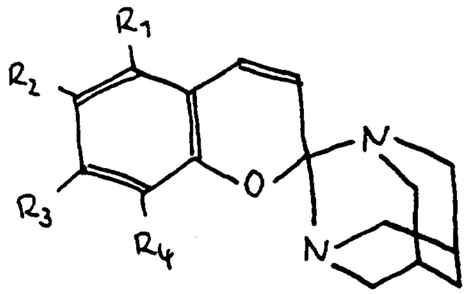

wobei die Reste

| | |
|---|---|
| $R_1$ - $R_4$ : | ein Substituent aus der Reihe H, Alkyl, Naphthyl, OH, Alkoxy ($C_1$ - $C_4$), Halogen, Alkylamino, Dialkylamino, Cyan und Trifluormethyl sind, oder |
| $R_1$ und $R_2$ oder $R_3$ und $R_4$ oder $R_2$ und $R_3$ | Bestandteil eines ankondensierten aromatischen oder heteroaromatischen Ringes oder eines Alkanringes mit 4-8 C-Atomen sind, |

als photochrome Substanz.

**5.** Verwendung von Spiro[adamantan-2,2'-(2H)-4'-chlorpyrido[4,3-e]-1',3'-oxazin], Spiro[1,3-Dimethyladamantan-2,2'-4''-chlornaphtho[2,1-e]-(2H)-pyran], Spiro[5,7-Dimethyl-adamantan-2,2'-4''-chlornaphtho[2,1-e]-(2H)-pyran], Spiro[1,3-Diazaadamantan-2,2'-4''-chlornaphtho[2,1-e]-(2H)-pyran] als photochrome Substanz.

**Claims**

**1.** Application of adamantano spiro hetero aromatic pyrans

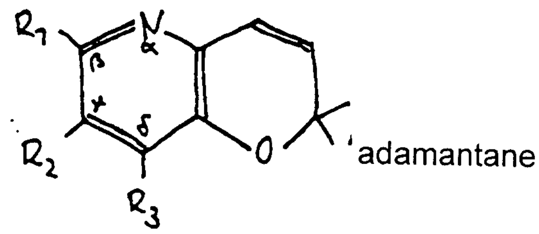

4 pyrido pyrans
(N-atom in α, β, γ or δ position)

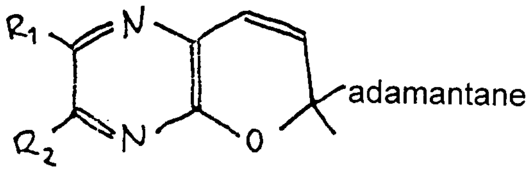

1 pyrazino pyran

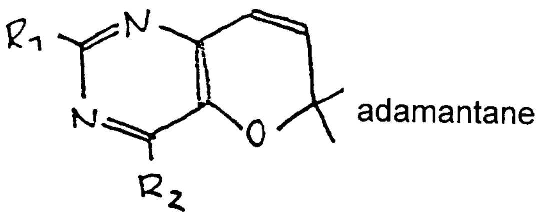

2 pyrimidino pyran
(N-atoms in α/γ or β/δ position)

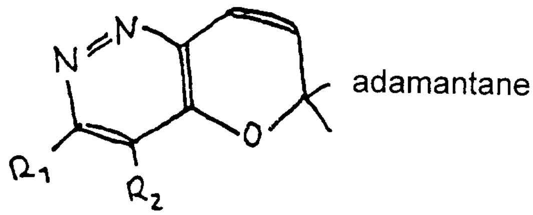

3 pyridazino pyran
(N-atoms α/γ or β/δ position)

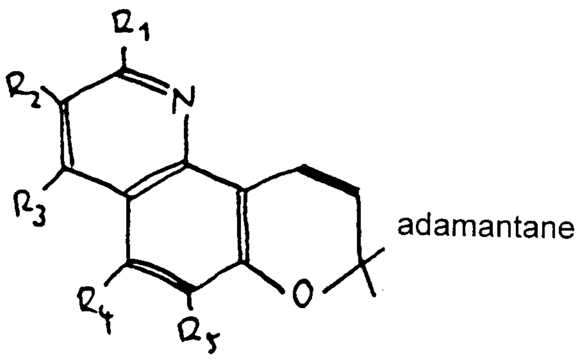

quinolino,
isoquinolino,
quinazolino,
quinoxalino,
phthalazino,
pteridino;
benzoquinolino and
phenazine pyrans

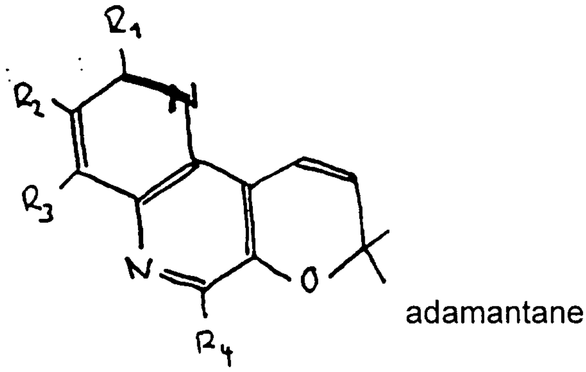

wherein:

| | |
|---|---|
| $R_1$ - $R_5$: | a substituent from the series H, alkyl, naphthyl, OH, alkoxy ($C_1$ - $C_4$), halogen, alkyl amino, di-alkyl amino, cyanogen and tri-fluoro methyl, or |
| $R_1$ and $R_2$ or $R_3$ and $R_4$ or $R_2$ and $R_3$ | are a constituent of a partially condensed aromatic or hetero aromatic ring or an alkane ring having 4 to 8 carbon atoms |

as photochromic substance.

**2.** Application of adamantano spiro (1.3) oxazine corresponding to the general formula:

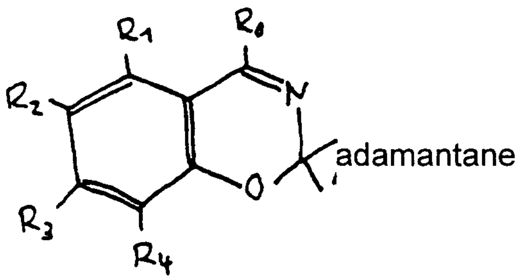

wherein:

| | |
|---|---|
| $R_0$ : | a substituent from the series H, O-alkyl, N-alkyl, |
| $R_1$ - $R_4$: | a substituent from the series H, alkyl, naphthyl, OH, alkoxy ($C_1$ - $C_4$), halogen, alkyl amino, di-alkyl amino, cyanogen and tri-fluoro methyl, or |
| $R_1$ and $R_2$ or $R_3$ and $R_4$ or $R_2$ and $R_3$ | are a constituent of a partially condensed aromatic or hetero aromatic ring or an alkane ring having 4 to 8 carbon atoms |

as photochromic substance.

**3.** Application of spiro[bi-cyclo[2,2,1]heptane-7.2'-4''-chloro naphtho[2,1-e]-(2H)-pyran] as photochromic substance.

**4.** Application of N-hetero cyclo pyrans and spiro benzo, naphtho, phenanthro oxazines, characterised in that they comprise, at the 2 position of the pyran or oxazine ring, a substituted adamantane, specifically 1,3-dimethyl or 1,3-di aza adamantane, corresponding to the general formulae:

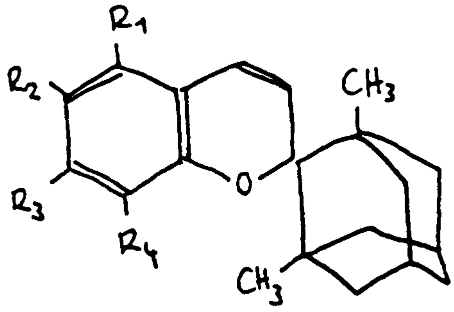

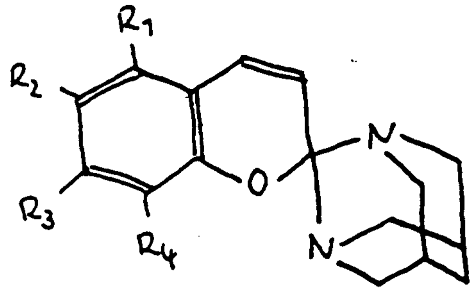

| | |
|---|---|
| $R_1$ - $R_2$: | are a substituent from the series H, alkyl, naphthyl, OH, alkoxy ($C_1$ - $C_4$), halogen, alkyl amino, di-alkyl amino, cyanogen and tri-fluoro methyl, or |
| $R_1$ and $R_2$ or $R_3$ and $R_4$ or $R_2$ and $R_3$ | are a constituent of a partially condensed aromatic or hetero aromatic ring or an alkane ring having 4 to 8 carbon atoms |

as photochromic substance.

**5.** Application of spiro[adamantane-2,2'-(2H)-4'-chloro pyrido[4,3-e]-1',3' oxazine], spiro[1,3-di-methyl adamantane-2,2'-4''-chloro naphtho[2,1-e]-(2H) pyran], spiro[5,7-di -methyl adamantane-2,2'-4''-chloro naphtho[2,1-e]-(2H)-pyran], spiro[1,3-di-aza adamantane-2,2'-4''-chloro naphtho[2,1-e]-(2H)-pyran] as photochromic substance.

**Revendications**

**1.** Utilisation d'adamantano spiro pyranes hétéro aromatiques

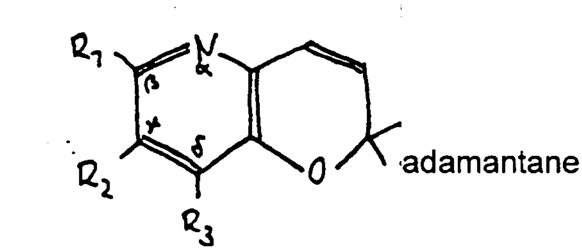

4 pyrido pyranes
(N-atomes en position α, β, γ ou δ)

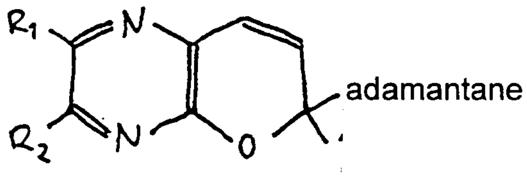

1 pyrazino pyrane

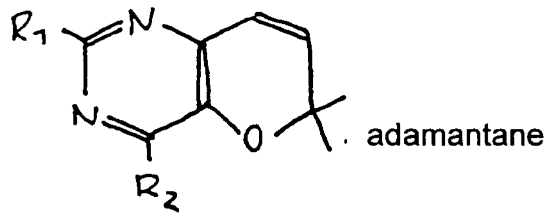

2 pyrimidino pyrane
(N-atomes en position α/γ ou β/δ )

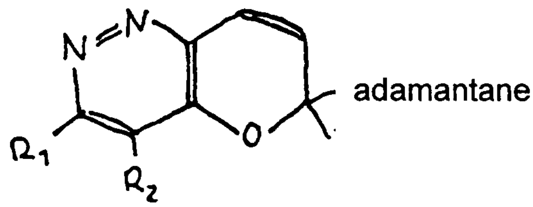

3 pyridazino pyrane
(N-atomes en position α/γ ou β/δ)

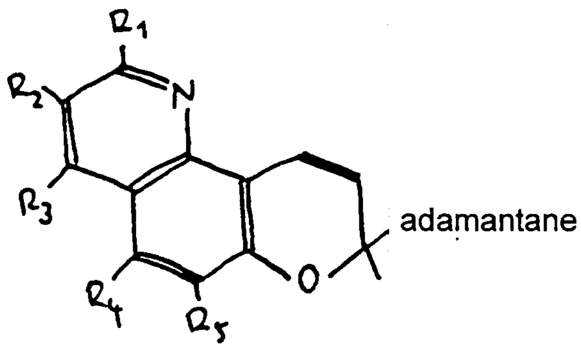

pyranes quinolinique,
isoquinolinique,
quinazolinique,
quinoxalinique,
phtalazinique,
pteridinique;
benzoquinolinque et
phenazinique

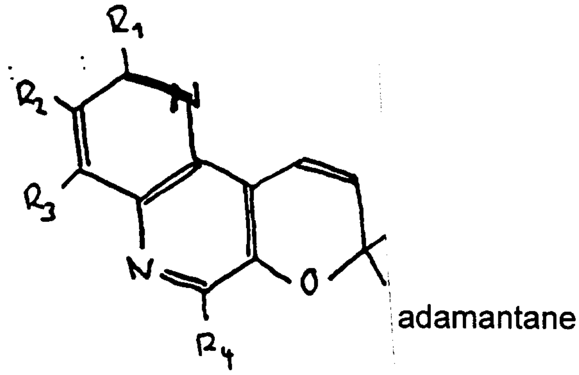

dans lequel:

| | |
|---|---|
| $R_1$ - $R_5$ : | un groupe substituant de la série H, alkyle, naphtyle, OH, alkoxy ($C_1$ - $C_4$), halogène, alkylamine, di-alkylamine, cyanogène et trifluoro méthyle, ou |
| $R_1$ et $R_2$ ou $R_3$ et $R_4$ ou $R_2$ et $R_3$ | sont un groupe constitutif d'un cycle aromatique ou hétéro aromatique partiellement condensé, ou d'un cycle alcanique à 4 à 8 atomes de carbone, |

en tant que substances photochromiques.

**2.** Utilisation de adamantano spiro (1.3) oxazine en correspondance avec la formule générale:

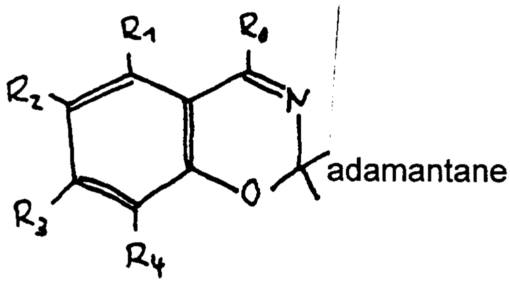

dans laquelle:

| | |
|---|---|
| $R_0$ : | un groupe substituant de la série H, O-alkyle, N-alkyle, |
| $R_1$ - $R_4$ : | un groupe substituant de la série H, alkyle, naphtyle, OH, alkoxy ($C_1$ - $C_4$), halogène, alkylamine, di-alkylamine, cyanogène et trifluoro méthyle, ou |
| $R_1$ et $R_2$ ou $R_3$ et $R_4$ ou $R_2$ et $R_3$ | sont un groupe constitutif d'un cycle aromatique ou hétéro aromatique partiellement condensé, ou un cycle alcanique à 4 à 8 atomes de carbone |

en tant que substances photochromiques.

**3.** Utilisation de spiro[bi-cyclo[2,2,1]heptane-7.2'-4''-chloro naphto[2,1-e]-(2H)-pyrane] en tant que substances photochromiques.

**4.** Utilisation de N-hétéro cyclo pyranes et spiro benzo, naphto, phenantro oxazines,
caractérisée en ce qu'ils comprennent, à la position 2 du cycle de pyrane ou d'oxazine, un adamantane substitué, notamment 1,3-diméthyle ou 1,3-di aza adamantane, en correspondance avec les formules générales:

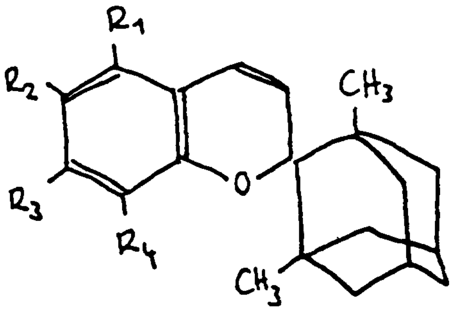 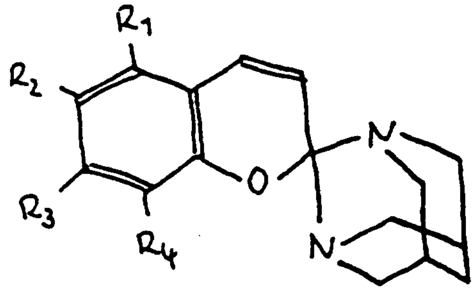

dans lesquelles les radicaux

| | |
|---|---|
| $R_1$ - $R_2$ : | sont un groupe substituant de la série H, alkyl, naphtyle, OH, alkoxy ($C_1$ - $C_4$), halogène, alkylamine, di-alkylamine, cyanogène et tri-fluoro méthyle, ou |
| $R_1$ et $R_2$ ou $R_3$ et $R_4$ ou $R_2$ et $R_3$ | sont un groupe constitutif d'un cycle aromatique ou hétéro aromatique partiellement condensé ou un cycle alcanique à 4 à 8 atomes de carbone. |

en tant que substances photochromiques.

**5.** Utilisation de spiro[adamantane-2,2'-(2H)-4'-chloro pyrido[4,3-e]-1',3' oxazine], spiro[1,3-di-méthyle adamantane-2,2'-4''-chloro naphto[2,1-e]-(2H)pyrane], spiro[5,7-di -méthyle adamantane-2,2'-4''-chloro naphto[2,1-e]-(2H)-pyrane], spiro[1,3-di-aza adamantane-2,2'-4''-chloro naphto[2,1-e]-(2H)-pyrane] en tant que substances photochromiques.